(19) Europäisches Patentamt
European Patent Office
Office européen des brevets

(11) Veröffentlichungsnummer : **0 360 084 B1**

(12) # EUROPÄISCHE PATENTSCHRIFT

(45) Veröffentlichungstag der Patentschrift : **19.11.92 Patentblatt 92/47**

(51) Int. Cl.$^5$ : **C07D 333/34,** C07D 409/12, A23L 1/226

(21) Anmeldenummer : **89116588.8**

(22) Anmeldetag : **08.09.89**

(54) **Heterocyclische Thioether, Verfahren zu ihrer Herstellung und ihre Verwendung.**

Die Akte enthält technische Angaben, die nach dem Eingang der Anmeldung eingereicht wurden und die nicht in dieser Patentschrift enthalten sind.

(30) Priorität : **21.09.88 DE 3831980**

(43) Veröffentlichungstag der Anmeldung : **28.03.90 Patentblatt 90/13**

(45) Bekanntmachung des Hinweises auf die Patenterteilung : **19.11.92 Patentblatt 92/47**

(84) Benannte Vertragsstaaten : **CH DE FR GB IT LI NL**

(56) Entgegenhaltungen :
**DE-A- 2 003 525**
**DE-A- 2 458 609**

(73) Patentinhaber : **HAARMANN & REIMER GMBH**
**Postfach 138**
**W-3450 Holzminden (DE)**

(72) Erfinder : **Emberger, Roland, Dr.**
**Bärenfang 4**
**W-3450 Holzminden (DE)**
Erfinder : **Güntert, Matthias, Dr.**
**Vogelsang 4**
**W-3450 Holzminden (DE)**
Erfinder : **Hopp, Rudolf, Dr.**
**Auf dem Gehrenkamp 28**
**W-3450 Holzminden (DE)**
Erfinder : **Köpsel, Manfred, Dr.**
**Schinkelstrasse 1**
**W-3450 Holzminden (DE)**
Erfinder : **Sand, Theodor, Dr.**
**Vogelsand 3**
**W-3450 Holzminden (DE)**
Erfinder : **Surburg, Horst, Dr.**
**Meiernberg 9**
**W-3450 Holzminden (DE)**
Erfinder : **Werkhoff, Peter, Dr.**
**Sieplerstrasse 24**
**W-3470 Höxter 1 (DE)**

(74) Vertreter : **Müller, Gerhard, Dr. et al**
**BAYER AG Konzernverwaltung RP**
**Patentabteilung**
**W-5090 Leverkusen 1 Bayerwerk (DE)**

**EP 0 360 084 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue heterocyclische Thioether, Verfahren zu ihrer Herstellung und ihre Verwendung als Aromastoffe.

Diskussion des Standes der Technik

Aus der DE-OS 20 03 525 ist bekannt, daß Bis(furyl)-mono- und -polysulfide als Aromastoffe verwendbar sind und Nahrungsmitteln einen Geschmack nach gebratenem Fleisch vermitteln können. Die der vorliegenden Erfindung nächstliegende Verbindung der DE-OS 20 03 525 ist das Bis-(2-methyl-3-furyl)-monosulfid. Wie Geschmackstests zeigen, weist diese Verbindung einen Fleischgeschmacksnote mit einer unangenehmen metallischen Beinote und einer fruchtigen Kopfnote auf, so daß diese Verbindung zum Verstärken und Abrunden von Fleischaromen ungeeignet ist.

Es wurden neue heterocyclishe Thioether der Formel

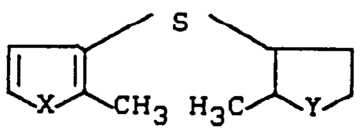

(I),

in der

X und Y unabhängig voneinander für ein Sauerstoffoder Schwefelatom stehen,

mit wertvollen Geschmacksstoffeigenschaften gefunden.

Die erfindungsgemäßen Verbindungen der Formel (I) werden durch Umsetzung von Verbindungen der Formel

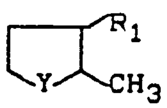

(II),

in der

Y die unter Formel (I) angegebene Bedeutung hat und

$R_1$ einen Tosylatrest oder ein Bromatom darstellt,

mit Thiolen der Formel

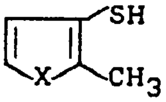

(III),

in der

X die unter Formel (I) angegebene Bedeutung hat,

erhalten. Bei der Umsetzung wird ein Gemisch der cis/trans-Isomeren der Thioether der Formel (I) erhalten; dieses Gemisch kann gegebenenfalls nach an sich bekannten Verfahren in seine Isomeren aufgetrennt werden.

Die Erfindung betrifft daher ein Verfahren zur Herstellung von heterocyclischen Thioethern der Formel (I), das dadurch gekennzeichnet ist, daß man Verbindungen der Formel (II) mit Verbindungen der Formel (III) in organischen Lösungsmitteln, vorzugsweise Alkoholen wie Methanol und Ethanol, bei Temperaturen von 0°C bis zur Siedepunkttemperatur des verwendeten Alkohols, vorzugsweise aber bei Raumtemperatur, in Gegenwart von Basen wie Natriumalkoholat oder -hydroxyd umsetzt und das bei der Umsetzung erhaltene Isomerengemisch gegebenenfalls in seine beiden Komponenten auftrennt.

Die Thiole der Formel (III) sind bekannt (siehe DE-OS 24 58 609), die Verbindungen der Formel (II) sind entweder bekannt (siehe DE-OS 19 32 800) oder nach bekannten Verfahren aus Ketonen der Formel

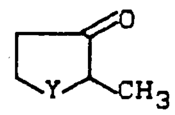

(IV),

in der

Y die unter Formel (I) angegebene Bedeutung hat,

erhältlich (siehe DE-OS 19 32 800; Arkiv för Kemi 31, 95 (1968)).

Die erfindungsgemäßen Verbindungen sind wertvolle Aromastoffe, die sich durch sehr niedrige Geschmacksschwellenwerte auszeichnen. Sie können sowohl in Form des bei der Herstellung anfallenden Isomerengemisches als auch in Form der einzelnen Isomeren verwendet werden.

Die Geschmacksbeschreibungen für einige typische Vertreter der erfindungsgemäßen heterocyclischen Thioether der Formel (I) lauten wie folgt:

cis-2-Methyl-3-(2-methyl-3-thienylthio)tetrahydrothiophen:
Leber, fleischig, krautig, pilzig

trans-2-Methyl-3-(2-methyl-3-thienylthio)-tetrahydrothiophen:
Mundfülle, Leber, Blutnote, fleischig, Braten

2-Methyl-3-(cis-2-methyl-3-tetrahydrothienylthio)-furan:
Geschmacksfülle, fleischig, Leber, Blutnote, Nuß, schwach pilzig

2-Methyl-3-(trans-2-methyl-3-tetrahydrothienylthio)-furan:
fleischig, Braten, Nuß, fettig, Spargel, schwefelig

Mit ihrem spezifischen Geschmack in Richtung Fleisch wirken die erfindungsgemäßen Verbindungen der Formel (I) in Fleischaromakompositionen geschmacksverstärkend und -abrundend. Darüber hinaus bewirken die Verbindungen der Formel (I) in anderen Aromakompositionen, insbesondere in Nuß- und Haselnußaromen, eine Abrundung des Aromas und einen erhöhte Geschmacksfülle.

Die unter Verwendung der erfindungsgemäßen Verbindungen hergestellten Aromakompositionen können im gesamten Nahrungsmittel- und Genußmittelbereich sowie in Tierfutter eingesetzt werden. Insbesondere sind sie geeignet für Nougatmassen, Fettmassen, Backwaren, Extrusionsprodukte, Fertiggerichte, Fleisch- und Wurstprodukte, Suppen, Soßen, Gemüsekonserven und alle Arten von industriell gefertigtem Tierfutter.

Die erfindungsgemäßen heterocyclischen Thioether werden in Mengen von 50 ppb bis 1 %, vorzugsweise 0,7 ppb bis 100 ppm, bezogen auf das verzehrfertige Nahrungsmittel verwendet.

Bei den in den Beispielen verwendeten %-Angaben handelt es sich um Gewichts-%.

## Beispiel 1

Die Lösung von 2,72 g (10 mmol) 2-Methyl-3-p-toluolsulfonyloxytetrahydrothiophen in 50 ml Ethanol wird bei Raumtemperatur unter Rühren und in einer Stickstoffatmosphäre zu einer Lösung von 1,14 g (10 mmol) 2-Methyl-3-furylthiol und 0,54 g (10 mmol) Natriummethylat in 50 ml Ethanol getropft. Nach beendeter Zugabe wird das Reaktionsgemisch 24 Stunden bei Raumtemperatur gerührt. Dann wird das Ethanol abdestilliert, der Rückstand mit Wasser versetzt und mit Ether extrahiert. Die vereinigten Etherextrakte werden in üblicher Weise aufgearbeitet.

Das auf diese Weise erhaltene farblose Öl wird durch Flüssigchromatographie an Kieselgel (Lösungsmittel: Hexan/Essigester 40:1) gereinigt. Das auf diese Weise erhaltene Gemisch der isomeren cis/trans-Verbindungen des 2-Methyl-3-(2-methyl-3-tetrahydrothienylthio)-furans wird durch präparative Kapillar-Gaschromatographie in die beiden Isomeren 2-Methyl-3-(cis-2-methyl-3-tetra-hydrothienylthio)-furan und 2-Methyl-3-(trans-2-methyl-3-tetrahydrothienylthio)-furan getrennt.

Sowohl das Isomerengemisch als auch die beiden Isomeren stellen farblose stark riechende Flüssigkeiten dar. Die Isomeren wurden anhand ihrer IR-, NMR- und ihrer Massenspektren identifiziert.

## Beispiel 2

Es wurde wie in Beispiel 1 beschrieben gearbeitet, nur wurde anstelle des 2-Methyl-3-furylthiols 10 mmol 2-Methyl-3-thienylthiol eingesetzt. Die Reinigung des Rohproduktes wurde an Kieselgel mit Hexan/Essigester 50:1 vorgenommen.

Sowohl das Isomerengemisch als auch die reinen Isomeren, cis-2-Methyl-3-(2-methyl-3-thienylthio)-tetrahydrothiophen und trans-2-Methyl-3-(2-methyl-3-thienylthio)-tetrahydrothiophen, stellen farblose stark reichende Flüssigkeiten dar. Die Verbindungen wurden anhand ihrer IR-, NMR- und Massenspektren identifiziert.

Beispiel 3

Eine Fleischaromakomposition wird durch Mischen folgender Bestandteile hergestellt:

| | |
|---|---|
| 50:50 Mischung von Na-Inosinat und Na-Guanilat | 1 |
| Mononatriumglutamat | 19 |
| Milchsäure, sprühgetrocknet | 30 |
| Pflanzenproteinhydrolysat (Type RFB der Food Ingredients Specialities) | 350 |
| Süßmolkenpulver | 100 |
| Speisesalz | 500 |
| | 1000 |

Eine 1 %ige wäßrige Lösung dieses Aromas dient als Kontrollprobe.

Werden zu der Kontrollprobe 200 ppb cis-2-methyl-3-(2-methyl-3-thienylthio)-tetrahydrothiophen gegeben, so wird der Geschmack von einer Testgruppe im Vergleich zur Kontrollprobe als kräftiger, fleischiger beurteilt.

Wird die Kontrollprobe mit 60 ppb des entsprechenden trans-Isomeren versetzt, so wird der Geschmack der wäßrigen Lösung im Vergleich zur Kontrollprobe als wesentlich voller, fleischiger, mit ausgeprägterer Bratennote beschrieben.

Wird die Kontrollprobe mit 12 ppb 2-Methyl-3-(cis-2-methyl-3-tetrahydrothienylthio)-furan versetzt, so wird der Geschmack der wäßrigen Lösung im Vergleich zur Kontollprobe als deutlich voller und wesentlich fleischiger in Richtung gekochte Leber beschrieben.

Wird die Kontrollprobe mit 20 ppb des entsprechenden trans-Isomeren versetzt, so wird der Geschmack der wäßrigen Lösung im Vergleich zur Kontrollprobe als voller und fleischiger beschrieben. Der Aromacharakter geht in Richtung Fleischbrühe mit einer Lebernote.

Beispiel 4

Eine Haselnußaromakomposition wird durch Mischen folgender Bestandteile hergestellt:

| | |
|---|---|
| Vanillin | 30 |
| Benzaldehyd | 10 |
| Furfurol | 5 |
| 2-Ethyl-3,5(3,6)-dimethylpyrazin | 5 |
| 2-Methyl-3-ethylpyrazin | 5 |
| Resorcindimethylether | 50 |
| Propylenglykol | 895 |
| | 1000 |

Eine 15 ppm dieser Komposition enthaltende 5 %ige wäßrige Saccharoselösung dient als Kontrollprobe.

Wird die Kontrollprobe mit 2 ppb 2-Methyl-3-(cis-2-methyl-3-tetrahydrothienylthio)-furan versetzt, so wird der Geschmack der wäßrigen Lösung voller und typischer in Richtung gerösteter Haselnuß im Vergleich zur Kontrollprobe.

Wird die Kontrollprobe mit 4 ppb des entsprechenden trans-Isomeren versetzt, so wird der Geschmack der wäßrigen Lösung voller und geht mehr in Richtung fettiger Haselnuß als bei der Kontrollprobe.

## Patentansprüche

1. Heterocyclische Thioether der Formel

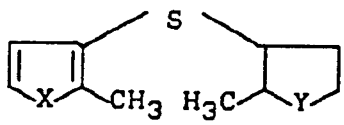

(I),

in der

X und Y unabhängig voneinander für ein Sauerstoff- oder Schwefelatom stehen.

2. Verfahren zur Herstellung von heterocyclischen Thioethern der Formel

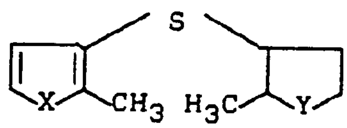

(I),

inder

X und Y unabhängig voneinander für ein Sauerstoff- oder Schwefelatom stehen, dadurch gekennzeichnet, daß man Verbindungen der Formel

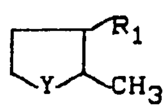

(II),

in der

Y die unter Formel (I) angegebene Bedeutung hat und

$R_1$ ein Tosylatrest oder ein Bromatom ist,

mit Thiolen der Formel

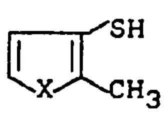

(III),

in der

X die unter Formel (I) angegebene Bedeutung hat, umsetzt.

3. Verwendung der heterocyclischen Thioether nach Anspruch 1 als Aromastoffe.

## Claims

1. Heterocyclic thioethers of the formula

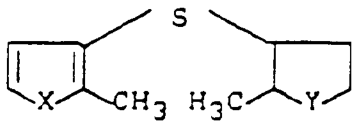

(I)

in which

X and Y independently of one another represent an oxygen or sulphur atom.

2. Process for the preparation of heterocyclic thioethers of the formula

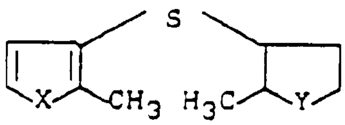

(I)

in which

X and Y independently of one another represent an oxygen or sulphur atom, characterized in that compounds of the formula

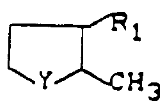

(II)

in which

Y has the meaning indicated under formula (I) and
$R_1$ is a tosylate radical or a bromine atom,
are reacted with thiols of the formula

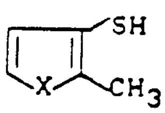

(III)

in which

X has the meaning indicated under formula (I).

3. Use of the heterocyclic thioethers according to Claim 1 as flavourings.

**Revendications**

1. Thio-éthers hétérocycliques de formule

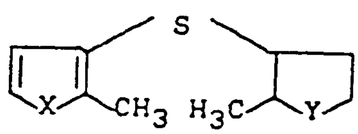

(I)

dans laquelle

X et Y représentent, indépendamment l'un de l'autre, un atome d'oxygène ou un atome de soufre.

2. Procédé de production de thio-éthers hétérocycliques de formule

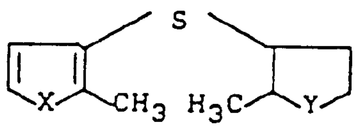

(I)

dans laquelle
X et Y représentent, indépendamment l'un de l'autre, un atome d'oxygène ou un atome de soufre, caractérisé en ce qu'on fait réagir des composés de formule

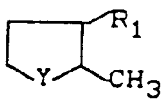

(II)

dans laquelle
Y a la définition indiquée pour la formule (I) et
$R_1$ est un reste tosylate ou un atome de brome,
avec des thiols de formule

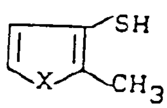

(III)

dans laquelle
X a la définition indiquée pour la formule (I).

3. Utilisation des thio-éthers hétérocycliques suivant la revendication 1 comme arômes.